# EUROPEAN PATENT SPECIFICATION

(11) **EP 2 863 917 B1**
(45) Date of publication and mention of the grant of the patent: **04.10.2017**
(21) Application number: 13733033.8
(22) Date of filing: 21.06.2013
(51) Int. Cl.: A61K 31/513, A61K 31/52, A61P 25/00

(54) **THYMIDINE AND PURINES FOR PREVENTING NEURAL TUBE DEFECTS**
THYMIDINE UND PURINE ZUR VORBEUGUNG VON NEURALROHRDEFEKTEN
THYMIDINE ET PURINES POUR LA PRÉVENTION DES DÉFAUTS DU TUBE NEURAL

(30) Priority: 22.06.2012 GB 201211135
(43) Date of publication of application: 29.04.2015
(73) Proprietor: UCL Business PLC, London W1T 4TP (GB)
(72) Inventor: GREENE, Nicholas, London Greater London WC1N 1EH (GB); COPP, Andrew, London Greater London WC1N 1EH (GB)
(74) Representative: J A Kemp
(86) International application number: PCT/GB2013/051640
(87) International publication number: WO 2013/190325

(56) References cited:
- WO-A1-2012/148998
- FLEMING ANGELEEN ET AL: "Embryonic folate metabolism and mouse neural tube defects", SCIENCE (WASHINGTON D C), vol. 280, no. 5372, 26 June 1998 (1998-06-26), pages 2107-2109, XP8163981, ISSN: 0036-8075
- BANNIGAN J G: "THE EFFECTS OF 5 BROMODEOXYURIDINE ON FUSION OF THE CRANIAL NEURAL FOLDS IN THE MOUSE EMBRYO", TERATOLOGY, vol. 32, no. 2, 1985, pages 229-240, XP8163985, ISSN: 0040-3709
- COPP A J ET AL: "Embryonic mechanisms underlying the prevention of neural tube defects by vitamins", MENTAL RETARDATION AND DEVELOPMENTAL DISABILITIES RESEARCH REVIEWS 1998 US, vol. 4, no. 4, 1998, pages 264-268, XP8163994, ISSN: 1080-4013
- WLODARCZYK B J ET AL: "Spontaneous neural tube defects in splotch mice supplemented with selected micronutrients", TOXICOLOGY AND APPLIED PHARMACOLOGY, ACADEMIC PRESS, AMSTERDAM, NL, vol. 213, no. 1, 15 May 2006 (2006-05-15), pages 55-63, XP024896228, ISSN: 0041-008X, DOI: 10.1016/J.TAAP.2005.09.008 [retrieved on 2006-05-15]

## Description

### Field of the Invention

The present invention relates to the use of nucleotides and their precursors for preventing or treating neural tube defects.

### Background of the Invention

Neural tube defects (NTDs) are severe malformations of the central nervous system, occurring in 0.5 to 2 per 1000 pregnancies. Among 120 million births each year world-wide (i.e. approximately 20 births per year per 1000 population, in a world population of 6 billion), around 120,000 cases of NTD occur, either born or terminated after prenatal diagnosis. NTDs result when the embryonic neural tube, the embryonic precursor of the brain and spinal cord, fails to close at weeks 3-4 of gestation. As pregnancy progresses, exposure to the amniotic fluid environment leads to neurodegeneration so that, by birth, the exposed brain and/or spinal cord are totally degenerate. Fetuses with brain NTDs (e.g. anencephaly) are stillborn while those with spinal NTDs (e.g. myelomeningocele/open spina bifida) often survive, but are neurologically impaired below the lesion. Children with myelomeningocele often lack sensation, cannot stand/walk, and are incontinent of urine and faeces. Associated conditions include hydrocephalus and vertebral deformities. In humans, neural tube closure is completed by day 28 of gestation and there is no effective treatment if closure fails, in utero surgery offering possible palliation but not remediation of the defects. Therefore, primary prevention is the optimal approach to reduce the burden of NTDs.

Prenatal diagnosis enables termination of NTD pregnancies in the UK and other Western countries, but this is not universal. Babies with NTDs continue to be born in the majority of countries. Primary prevention by folic acid (FA) offers a possible universal solution to this problem: randomised clinical trials in the UK and Hungary showed that FA administered early in pregnancy can prevent up to 70% of NTD recurrences [Wald et al (1991) Lancet 338: 131-137] with an apparent preventive effect also on first occurrence [Czeizel et al (1992) N Engl J Med 327: 1832-1835].

If a prospective mother has suffered a previous affected pregnancy the recurrence risk for NTDs is more than 10-fold higher and these women are advised to take folic acid at a higher dose of 5 mg/day which reduces risk by an estimated 70%, based on clinical trial data. Nevertheless, evidence from clinical trials, reports of trial data. Nevertheless, evidence from clinical trials, reports of multiple affected pregnancies despite use of folic acid and epidemiological studies all show that a subset of NTDs are not responsive to folic acid [Blom et al (2006) Nat Rev Neurosci 7: 724-731].

Concerns about the level of voluntary uptake of FA supplementation prior to and during early pregnancy led the USA to fortify bread flour with FA, with a subsequent 26% reduction in the prevalence of NTDs [Mersereau et al. (2004) MMWR 53(17): 362-365]. Recently, other countries (e.g. Canada, Chile) have also reported reduced prevalence of NTDs following food fortification although, in all cases, the frequency of NTDs has remained in the 0.5-1.5 per 1000 range [Eichholzer et al (2006) Lancet 367: 1352-1361]. Hence, NTDs remain common birth defects, even post-FA fortification. A recent study suggests that there is no longer a benefit of additional supplement usage in the US population, suggesting that simply increasing dosage may not achieve significant further protection [Mosley et al. (2009) Am J Epidemiol 169: 9-17]. The consensus has therefore been reached that a proportion of NTDs cannot be prevented by current strategies and additional therapies are required to further reduce the incidence of these birth defects. Novel therapies are needed to improve NTD prevention, by encompassing folate-resistant cases which currently cannot be prevented.

### Summary of the Invention

The present inventors have found that nucleosides or nucleotides in combination with purines may be used to prevent neural tube defects, even in some individuals who are not responsive to treatment with folic acid.

Accordingly, the present invention provides thymidine or dTMP for use in a method of preventing or prophylactically treating a neural tube defect, wherein the thymidine or dTMP is administered in combination with one or more purine molecules selected from adenine, guanine, adenosine, guanosine, AMP and GMP.

Preferably the purine molecule is selected from adenine, adenosine and AMP or from guanine, guanosine and GMP, such as adenine or GMP.

The thymidine or dTMP and the purine molecule may be administered to the female mammal in a single composition. The female mammal may also be administered folic acid or folate. Administration may be oral administration.

The invention also provides compositions for use in the methods of the invention, specifically a composition comprising thymidine and/or dTMP for use in a method of preventing or prophylactically treating a neural tube defect, wherein said composition further comprises one or more purine molecules selected from adenine, guanine, adenosine, guanosine, AMP and GMP. Such a composition may be administered to a female mammal that is pregnant or that may become pregnant. Such a composition may further comprise folic acid and/or folate.

The methods, agents and uses of the invention may be for preventing miscarriage or for preventing or treating recurrent miscarriage. The female mammal may be non-responsive to treatment with folate or folic acid. The female mammal may have previously given birth to an offspring having a neural tube defect. The female mammal may have previously miscarried an offspring having a neural tube defect. The female mammal may have been diagnosed as susceptible to or at risk of producing offspring having a neural tube defect.

### Detailed Description of the Drawings

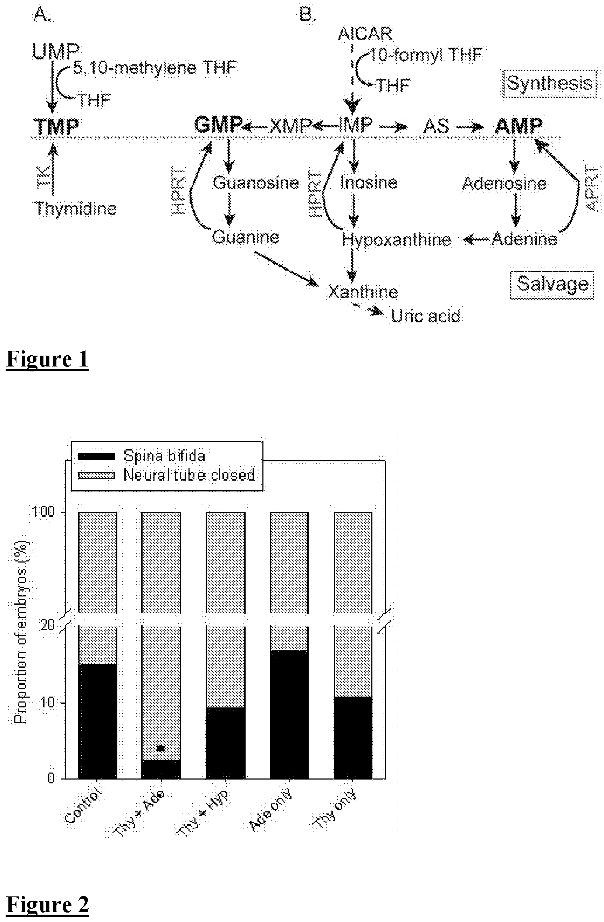

Figure 1 shows a summary of nucleotide biosynthesis and salvage pathways. Folates, based on a tetrahydrofolate (THF) backbone, contribute one-carbon units for de novo synthesis of (A) pyrimidine and (B) purine nucleotide precursors (in bold; TMP, AMP and GMP). A salvage reaction mediated by thymidine kinase (TK) also allows usage of thymidine as the nucleoside precursor of TMP, while the purine bases adenine and hypoxanthine can be salvaged by the action of adenine phosphoribosyltransferase (APRT) and hypoxanthine phosphoribosyltransferase (HPRT), respectively.
Figure 2 shows the proportion of homozygous *curly tail* (*ct*/*ct*) embryos having spina bifida. Controls = untreated ct/ct embryos, 172 animals. Groups of ct/ct obtained after maternal treatment with thymidine alone (Thy, 74 animals), adenine alone (Ade, 60 animals), Thymidine and adenine (Thy + Ade, 88 animals) or thymidine + hypoxanthine (Thy + Hyp, 64 animals).

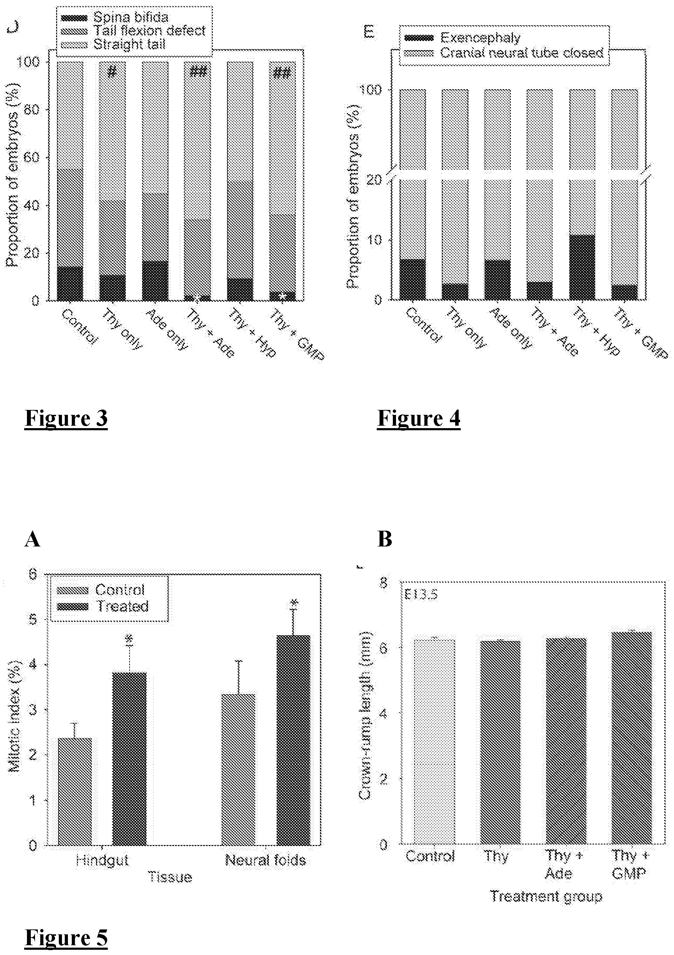

Figure 3 shows the proportion of the ct/ct embryos having spina bifida or tail flexion defect.
Figure 4 shows the frequency of exencephaly (cranial NTDs) among genetically predisposed *curly tail* embryos.
Figure 5 shows the stimulation of cellular proliferation by supplemental nucleotides. (A) Mitotic index determined in the hindgut and neural folds of embryos treated with thymidine + adenine compared to controls. (B) Fetal size at E13.5 as determined by measurement of crown-rump length compared between control litters and those exposed to supplemental nucleotides at E7.5 - 10.5, data expressed as mean ±SEM, number of fetuses analysed n = 22 controls, 13 Thy only, 39 Thy + Ade, 21 Thy+GMP.

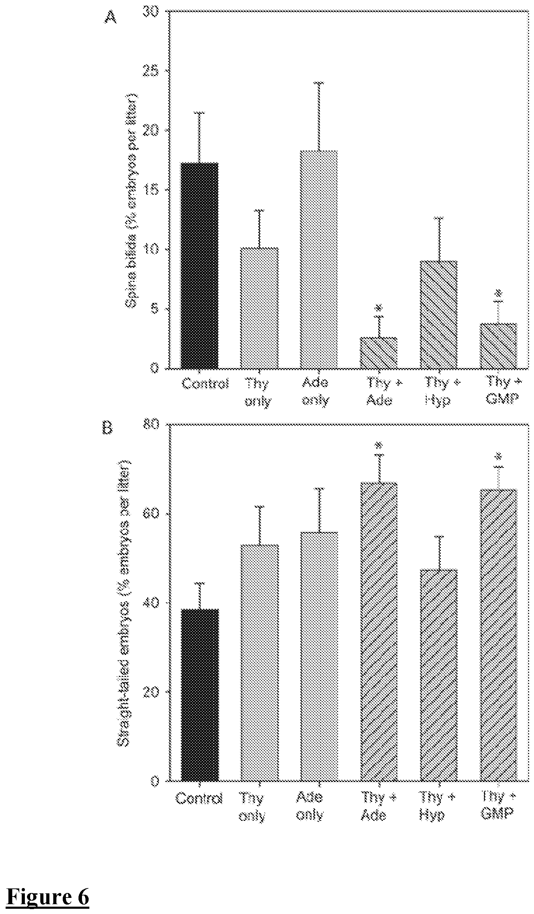

Figure 6 shows the frequency of spina bifida and straight tails among litters of *curly tail* embryos supplemented with nucleotide precursors. Offspring of mice treated with vehicle only (control), a single compound (thymidine only or adenine only) or combinations of compounds were analysed. Among litters of mice treated with Thy + Ade or Thy + GMP, the proportion of (A) embryos affected by spina bifida was significantly lower than among control litters, while (B) in the same treatment groups the proportion of unaffected, straight tail embryos, was significantly higher than among controls (*p<0.05; One Way ANOVA). Data are shown as mean% embryos within litter ±STEM.

### Detailed Description of the Invention

The present invention is based on the use of thymidine (the nucleoside precursor of the nucleotide dTMP) or dTMP itself to prevent neural tube defects (NTDs) in combination with one or more purine molecules. The thymidine or dTMP may be used in combination with other agents, including other agents for the prevention of NTDs such as folic acid. Preferred combinations include a combination of thymidine or dTMP with adenosine.

### Thymidine

In accordance with the present invention, thymidine or a related molecule is used. Thymidine, also known as deoxythymidine, is a nucleoside composed of deoxyribose joined to the pyrimidine base thymine. The compositions of the present invention may use the nucleoside thymidine.

Thymidine can be phosphorylated with one, two or three phosphoric acid groups to form dTMP (deoxythymidine monophosphate), dTDP (deoxythymidine diphosphate) or dTTP (deoxythymidine triphosphate). The methods of the present invention may use a phosphorylated form of thymidine, namely dTMP.

The present invention therefore uses thymidine or a related molecule. The related molecule is another molecule based on a thymine base, i.e. dTMP.

The thymidine or related molecule may be used in a pure form such as a pure crystalline form. Isolated forms of the thymidine or related molecule are typically used. Any active form of thymidine or any active thymidine related molecule may be used in the methods of the invention.

The thymidine or related molecule may be in a physiologically acceptable salt form, such as an alkali metal or alkaline earth metal salt. A suitable salt may be a sodium salt.

The invention typically uses one molecule selected from thymidine and a related molecule as described herein. However, the compositions of the invention may alternatively use more than one such molecule. That is, the compositions of the invention may use two or more thymidine or related molecules. Any description herein of a use of thymidine or a related molecule is intended to encompass the use of two or more such molecules. For example, both thymidine and dTMP may be used in accordance with the present invention.

### Purine Molecules

In accordance with the present invention, one or more purine molecules are used. One or more purines according to the claims are used in combination with thymidine or a related molecule. The thymidine or related molecule may be thymidine or dTMP. The thymidine or a related molecule and the purine(s) may be used in combination in any of the therapeutic or prophylactic methods or uses described herein.

Where a purine molecule or the purine molecule is mentioned herein, it may be understood that one, two or more such purine molecules may be used.

The purine(s) may each independently be any purine molecule described herein, such as any purine molecule shown in Figure 1.

For example, the purine molecule may be a purine base, namely adenine or guanine.

The purine molecule may be a purine nucleoside, namely guanosine or adenosine.

The purine molecule may be a purine nucleotide, namely GMP or AMP.

Particularly preferred are combinations of thymidine or dTMP with a purine molecule that is adenine or adenosine or AMP.

Also preferred are combinations of thymidine or dTMP with a purine molecule that is guanine or guanosine or GMP.

The purine molecule may be used in a pure form such as a pure crystalline form. Isolated forms of the purine are typically used. Any active form of the purine molecule may be used in the methods of the invention.

The purine molecule may be in a physiologically acceptable salt form, such as an alkali metal or alkaline earth metal salt. A suitable salt may be a sodium salt.

The invention typically uses one purine molecule as described herein. However, the methods of the invention may alternatively use more than one such molecule. That is, the methods of the invention may use two or more different purine molecules. Any description herein of a use of a purine is intended to encompass the use of two or more such purine molecules.

### Other combination treatments

The compositions of the invention may be used in combination with other compositions to prevent NTDs.

For example, a composition of the invention may be used in combination with administration of folic acid or folate. That is, an individual to be treated in accordance with the present invention may additionally be administered folic acid or folate.

The folic acid or folate may be administered in addition to thymidine or a related molecule as described herein such as dTMP. The folic acid or folate may be administered in addition to thymidine or a related molecule and one or more purines as described herein. Thus, in the present invention, a combination of (a) thymidine or a related molecule, (b) one or more purines and (c) folic acid or folate may be used. These three components may be administered together in a single composition. The components may be administered separately or sequentially as part of a combined treatment in two or more compositions. For example, thymidine or a related molecule and one or more purines may be administered as described herein. The same individual may also be administered folic acid or folate. The same individual may also be administered inositol as described below.

A composition of the invention may be used in combination with administration of inositol. That is, an individual to be treated in accordance with the present invention may additionally be administered inositol. The inositol is preferably D-chiro-inositol or myo-inositol.

The inositol may be administered in addition to thymidine or a related molecule as described herein such as dTMP. The inositol may be administered in addition to thymidine or a related molecule and one or more purines as described herein. Thus, in the present invention, a combination of (a) thymidine or a related molecule, (b) one or more purines and (c) inositol may be used. These three components may be administered together in a single composition. The components may be administered separately or sequentially as part of a combined treatment in two or more compositions. For example, thymidine or a related molecule and one or more purines may be administered as described herein. The same individual may also be administered inositol. The same individual may also be administered folic acid or folate as described above.

### Compositions

The present invention provides compositions for use in the methods of the invention. That is, the invention provides compositions for administration to a female mammal in need therefore in order to e.g. prevent a NTD in a fetus or prevent a consequence of carrying a fetus with an NTD such as prevention of a miscarriage.

Compositions of the invention will comprise thymidine or a related molecule as described herein. Compositions of the invention further comprise one or more purine molecules as described herein. Compositions of the invention may further comprise one or more additional components intended for administration as part of the invention, such as folic acid, folate or inositol.

Compositions of the invention may be pharmaceutical compositions which further comprise a pharmaceutically acceptable carrier or diluent. The pharmaceutical silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with at least one of phenylacetate, phenylbutyrate and other related molecule, a pharmaceutically acceptable carrier, e.g. sterile water, olive oil, ethyl oleate, glycols, e.g. propylene glycol, and if desired, a suitable amount of lidocaine hydrochloride.

Compositions of the invention may be provided in the form of a nutritional supplement. Various nutritional supplements exist for use prior to and/or during pregnancy. Such a supplement may be formulated to include the components required by the present invention, such as thymidine or a related molecule and optionally one or more purine molecules. Preferably the amounts of said components are suitable to provide the components in a daily dosage as described herein. That is, preferably such a nutritional supplement provides said components in a unit dosage form intended to provide the dosages described above to the female animal using the nutritional supplement.

### Neural Tube Defects

The present invention relates generally to the prevention of neural tube defects (NTDs).

Where an NTD is mentioned herein, the NTD may be a brain NTD such as anencephaly. The NTD may be an NTD that leads to miscarriage or to the fetus being stillborn. The NTD may be a spinal NTD such as myelomeningocele or open spina bifida. The NTD may be an open NTD wherein the brain and/or spinal cord are exposed at birth through a defect in the skull or vertebrae. Examples of open NTDs include spina bifida, myelomeningocele and anencephaly. The NTD may be a closed NTD wherein the spinal defect is covered by skin. Examples of closed NTDs include lipomyelomeningocele, lipomeningocele, and tethered cord. The NTD may be spina bifida occulta (SBO) in which there is a typically benign (or non-symptom-causing) bony change in one or more vertebrae, but not involving the nerves within the spinal column.

In accordance with the present invention, the NTD may be or may comprise any one or more neural tube disorders selected from spina bifida, spina bifida anterior, spina bifida aperta, spina bifida cystica, spina bifida occulta, spina bifida posterior, anencephaly, exencephaly, meningomyclocele, encephalocele, lipomyelomeningocele, lipomeningocele, and tethered cord.

The NTD may be a brain NTD such as anencephaly. The NTD may be a spinal NTD such as myelomeningocele or open spina bifida. The present invention may be used to prevent such a NTD or to prevent or reduce the symptoms of such a NTD. For example, the present invention may be used to prevent or reduce the severity of hydrocephalus, vertebral deformities, incontinence or any other symptom of a neural tube defect.

### Treatment and Prevention

The present invention relates to the prevention or prophylactic treatment of neural tube defects (NTDs). The invention also relates to the prevention of miscarriage, such as the prevention or treatment of recurrent miscarriage. For example, the invention relates to the prevention of miscarriage caused by NTD in a fetus.

The present invention provides compositions for treatment and prevention as discussed herein. It will be appreciated that a description herein of a method of treatment or prevention comprising administering one or more agents to an individual will also encompass the equivalent use of the one or more agents in the manufacture of a medicament for use in said prevention or treatment, and the one or more agents for use in such a method of treatment or prevention.

The present invention is broadly applicable to therapeutic methods and is relevant to prophylactic and/or therapeutic treatments. It is to be appreciated that all references herein to treatment may include curative, palliative and prophylactic treatment.

The or each molecule administered in accordance with the present invention is provided in a therapeutically or prophylactically effective amount. The exact amount or dosage can be determined according to various parameters such as the age, weight and condition of the subject to be treated; the likelihood or risk of an NTD pregnancy in that individual; the route of administration; and the required regimen.

For example, where thymidine or a related molecule such as dTMP is administered in accordance with the present invention, administration is preferably carried out over a period of at least 1 week, at least 2 weeks, at least 1 month, at least 2 months, at least 3 months or at least 4 months. Administration may be carried out regularly, for example on a weekly, daily, twice daily or more frequent basis.

Suitable dosages will depend upon the particular administration regimen used, but may typically be in an amount equivalent to 0.1 mg to 250 mg a day, such as 0.5 to 200 mg per day, 1 to 150 mg per day, 2 to 100 mg per day or 5 to 50 mg per day. A suitable dosage may be 2 to 20 mg per day. A suitable dosage may be at least 0.1 mg per day, at least 0.2 mg per day, at least 0.5 mg per day, or at least 1 mg per day, at least 2 mg per day, at least 5 mg per day or at least 10 mg per day. The dosage may be, for example, up to 5 mg per day, up to 10 mg per day, up to 20 mg per day, up to 50 mg per day, up to 100 mg per day, up to 125 mg per day or greater than 125 mg per day.

Where an additional molecule such as an a purine molecule as described herein is used in combination with thymidine or dTMP in accordance with the invention, the purine molecule may be administered in an amount that is independently selected from any of the amounts described above in relation to thymidine or dTMP.

Where the present invention makes use of multiple nucleotides or nucleosides, such as a combination of thymidine or a related molecule with a purine molecule, the total dosage of the nucleotide or nucleoside molecules being administered may be, for example, around 250 mg per day. The total dosage may be, for example, 10 to 500 mg per day, such as 20 to 400 mg per day, 50 to 300 mg per day or 100 to 250 mg per day.

Where folic acid or folate is used in combination with one or more other agents in accordance with the present invention, it is preferably used in an amount that is independently selected from any of the amounts described above in relation to thymidine or dTMP. For example, a treatment as described herein may be carried out in combination with administration of folic acid or folate in an amount of 0.1 to 5 mg per day. A typical dose of folic acid may be 0.1 mg per day, 0.25 mg per day, 0.5 mg per day, 1 mg per day, 2 mg per day or 5 mg per day. Where the treatment is of a female mammal with a prior history of NTD in a fetus such as a female with a prior miscarriage or recurrent miscarriage, a treatment as described herein may be carried out in combination with administration of folic acid in an amount of 4 mg to 5 mg per day. A preferred daily dose of folic acid or folate is 5 mg.

Where inositol is used in combination with one or more other agents in accordance with the present invention, it is preferably used in an amount that is independently selected from any of the amounts described above in relation to thymidine or dTMP. Preferably the dosage of inositol is greater than the dosage of thymidine. A suitable dosage for inositol may be, for example, up to 1g per day or up to 2g per day. A suitable dosage of inositol might be from 1g to 2g per day.

Where a method of the invention is a preventative method, the treatment described herein is preferably given to the female mammal prior to conception. If the female mammal is expected to conceive or if the female mammal may conceive, treatment as described herein may be commenced before the date of conception in order to prevent the occurrence of NTD in any fetus(es) resulting from that conception. Treatment may be commenced before conception. Treatment may be given or may be commenced up to a year before conception, up to six months before conception, up to three months before conception, up to two months before conception, up to one month before conception or up to two weeks before conception.

Treatment as described herein may additionally or alternatively be given after conception. Treatment as described herein is preferably started before the date of conception as discussed above and continued after the date of conception. Treatment as described herein may be started before the date of conception as discussed above and stopped after conception has been confirmed. Treatment as described herein may be started before the date of conception as discussed above and stopped during the first four weeks of pregnancy. Treatment as described herein may be started before the date of conception and continued to at least the fourth week after conception. Treatment as described herein may be started before the date of conception as discussed above and stopped at least four weeks, at least five weeks, at least six weeks, at least eight weeks, at least ten weeks, or at least twelve weeks after conception. Treatment as described herein may be started before the date of conception as discussed above and stopped during the first three months or four months of pregnancy, such as the first 12 weeks of pregnancy. Treatment may therefore be started up to three months prior to conception stopped at least four weeks, at least five weeks, at least six weeks, at least eight weeks, at least ten weeks, or at least twelve weeks after conception. Treatment as described herein may be started before the date of conception as discussed above and stopped during the first three months or four months of pregnancy, such as the first 12 weeks of pregnancy. Treatment may therefore be started up to three months prior to conception and continued for the first three months of pregnancy

Treatment as described herein may be given after conception but prior to the end of the fourth week of pregnancy. Treatment as described herein may be given immediately after conception, within the first week of pregnancy, within the first two weeks of pregnancy, within the first three weeks of pregnancy or within the first four weeks of pregnancy.

Treatment may be continued after conception for the first month, two months, three months or four months of pregnancy.

The invention relates to the administration of thymidine or a related molecule. The invention also relates to the administration of a purine molecule. The invention optionally also relates to the administration of folic acid and/or folate. The invention optionally also relates to the administration of inositol.

The invention thus relates to the administration of two or more different molecules; these molecules may be administered together in a single composition as described above. The molecules may be administered simultaneously but as part of two or more separate compositions. The molecules may be administered separately or sequentially as part of a combined therapy.

The molecules or compositions of the invention may be administered by any suitable route. Preferably administration is by oral, intravenous, intragastric, intraperitoneal or intravascular routes. Most preferred is oral administration. Oral administration may be achieved using a composition as described herein, such as a solid or liquid oral form. Oral administration may be achieved using a nutritional supplement as described herein that comprises thymidine or a related molecule and that optionally further comprises a purine molecule and/or folic acid and/or folate and/or inositol.

The molecules or compositions of the invention may be administered in one or more doses over the time of treatment. For example, the molecules to be administered may be provided in a sustained release composition. Such a composition may be administration preferably provides a daily dose of thymidine or a related molecule as described above. This may be achieved by, for example, carrying out a daily administration of the desired dose of each molecule to be administered. The molecules to be administered in accordance with the invention may therefore be provided in a unit dosage form. A suitable unit dosage form may be a formulation for oral administration such as a liquid or solid oral formulation. Such a unit dosage form may be intended for regular administration such as daily administration. Such a unit dosage form may comprise a daily dose of each molecule to be administered as described above. For example, such a unit dosage form may comprise a daily dosage of thymidine or a related molecule as described herein. Such a unit dosage form may further comprise a daily dosage of a purine molecule such as adenosine as described above. Such a unit dosage form may further comprise a daily dose of one or more other molecules of interest such as a daily dose of folic acid, folate or inositol as described above.

### Individual to be Treated

The individual to be treated in accordance with the present invention is typically a female mammal who is pregnant or a female mammal who may become pregnant. The individual is typically a human woman, but may alternatively be a non-human female mammal. The individual to be treated may be a farm animal, for example a cow or bull, sheep, pig, ox, goat or horse or may be a domestic animal such as a dog or cat.

The present invention may be used to prevent the occurrence of a NTD in a fetus. The present invention therefore relates to preventative or prophylactic treatments administered to the female mammal (e.g. woman) in order to prevent NTD in fetus(es) carried by the female mammal.

The female mammal may already be carrying the fetus at the time the treatment is administered. Treatment of the female mammal as described herein may therefore treat the deficiency or problem in a fetus being carried by that female mammal that would otherwise lead to an NTD in that fetus. Treatment of the female mammal as described herein may prevent the development of an NTD in a fetus already being carried by that female mammal.

The individual to be treated may therefore be a fetus carried by a pregnant mammal. The fetus is treated by administering a treatment as described herein to the mother carrying the fetus.

The female mammal may not be pregnant at the time of administration as described herein. Administration may be carried out prior to conception. The female mammal may be a female mammal that is at risk of pregnancy of that is expected to become pregnant such as a non-human female mammal that will be used for breeding purposes or a human female that is trying to become pregnant. Treatment of the female mammal as described herein may treat a deficiency or problem in the female mammal that might lead to a fetus being carried by that female mammal developing an NTD. Treatment of the female mammal as described herein may prevent the development of an NTD in a fetus carried by that female mammal when she does become pregnant.

The present invention may be used to prevent a miscarriage in a female mammal. The present invention therefore relates to preventative treatments intended to have a beneficial effect on the female mammal that is at risk of a miscarriage as well as a beneficial effect on the fetus(es) carried by that female animal.

The present invention may be used to treat a female mammal that suffers from recurrent miscarriage. If a prospective mother has suffered a previous affected pregnancy, the recurrence risk for NTDs is more than 10-fold higher than in the general population. The individual to be treated in the present invention may therefore be a female that has previously suffered a miscarriage, such as a miscarriage due to a NTD in a fetus. The individual to be treated may be a female that has previously suffered a pregnancy affected by a NTD in a fetus or a female that has previously given birth to an offspring having a NTD.

The individual to be treated may be a female mammal that has been diagnosed as at particular risk of producing a fetus with NTD. For example, the individual may be a female mammal with a family history of NTDs. The individual may be a female mammal with a genetic risk of producing a fetus with NTD. The individual may be a female mammal who has previously suffered a miscarriage due to NTD in the fetus or has previously had NTD diagnosed in a fetus or infant.

Around 30-50% of NTDs are not preventable by maternal folic acid treatment. For example, some NTDs in humans are associated with a genetically determined abnormality in folate metabolism in the fetus; these may correspond to folate-resistant NTDs. The curly tail (ct) mouse is a well-established model for human NTDs displaying pathogenic similarities, partial penetrance and significant effects of genetic modifiers and environmental factors. However, NTDs in ct/ct embryos are not preventable by supplementation with folic acid or related molecules [Van Straaten et al (2007) Hum Mol Genet 16: 2640-2646].

The individual to be treated may therefore be a female mammal that is resistant to or non-responsive to treatment with folic acid or folate. The individual may be a female mammal who has suffered a previous miscarriage or NTD affected pregnancy despite having received treatment with folic acid or folate prior to and/or during that earlier pregnancy. The individual to be treated may be a fetus that is resistant to or non-responsive to treatment with folic acid or folate.

### Examples

### Mouse model of neural tube defects

The curly tail (ct) mouse is considered a useful model for NTDs owing to a number of characteristics that resemble the corresponding defects in humans. These include a multifactorial etiology (with effects of both genetic and environmental factors), location of spina bifida lesions at the lumbosacral level, the presence of elevated alpha-fetoprotein in the amniotic fluid and a female excess among exencephalic embryos.

Approximately 15-20% of homozygous ct/ct embryos develop spina bifida due to failure of closure of the posterior neuropore (PNP) in the low spinal region, while a further 50% exhibit delayed closure resulting in tail flexion defects. The closure defects in ct/ct embryos are a mechanical consequence of excessive ventral curvature of the caudal region of the embryo. This, in turn, results from a dorso-ventral growth imbalance caused by a diminished cellular proliferation rate in the hindgut endoderm. In addition to spina bifida, cranial NTDs (exencephaly) also occur at low frequency among curly tail embryos (approximately 5%), but the cellular basis for these defects is not known.

### Mice and supplementation

Curly tail mice were maintained as a closed random-bred colony as described in Van Straaten et al (Anat Embryol 2001 203: 225-237).

Experimental litters were generated by overnight mating with the day of finding a copulation plug designated embryonic day (E) 0.5. Maternal supplementation was performed daily from E7.5-10.5. Treatment comprised intra-peritoneal injection with stock solutions (in sterile water), containing each reagent at a final concentration of 2 mg/ml, to give a dosage of 20 mg/kg.

Treatments were: control (water only), thymidine + adenine, thymidine + hypoxanthine, thymidine + GMP, thymidine only, adenine only (all reagents from Sigma). GMP was used instead of guanine owing to solubility considerations in preparing solutions for injection. Animal studies were carried out under regulations of the Animals (Scientific Procedures) Act 1986 of the UK Government, and in according with guidance issued by the Medical Research Council, UK in Responsibility in the Use of Animals for Medical Research (July 1993).

### Collection of embryos

Litters were dissected from the uterus, at E11.5-13.5, in Dulbecco's Modified Eagle's Medium containing 10% fetal calf serum and assessed for presence of neural tube defects (NTDs) under a light microscope. Any resorptions were recorded. Crown-rump length was measured using an eyepiece graticule.

Embryos were rinsed in phosphate buffered saline (PBS) and fixed in 4% paraformaldehyde for immunohistochemistry.

### Incorporation of nucleotides in utero and in embryo culture

Embryos were explanted at E9.5 and cultured for 24 hours in rat serum as described previously (Cockroft, In: Copp AJ, Cockroft DL, editors. Postimplantation Mammalian Embryos: A Practical Approach. Oxford: IRL Press; 1990. p. 15-40; Greene et al., Anat Embryol 2003; 206: 185-191), in the presence of [³H]-thymidine, [³H]-hypoxanthine or [³H]-adenine (at 1 or 2 µCi/ml). Genomic DNA was isolated and incorporation of [³H] determined by scintillation counting as described previously (Dunlevy et al., Brain 2007; 130: 1043-1049). DNA concentration was determined by NanoDrop.

### Proliferation analysis

Immunohistochemistry for phospho-histone H3 was performed on transverse 7 µm sections at the axial level of the closing neural folds (5-7 sections per embryo) in embryos at E10.5 (28-31 somites) as described previously (Gustavsson et al., Hum Mol Genet 2007; 16: 2640-2646).

Primary and secondary antibodies were anti-phospho-histone H3 (1:250, Millipore) and Alexa Fluor 488-conjugated anti-rabbit (1:500, Invitrogen). For nuclear staining, cells were incubated with Hoechst (1:2,000 in PBS). Fluorescent images were collected on an Axiophot microscope (Zeiss) with a DC500 camera (Leica), using FireCam software (Leica). Images were analysed using the Cell Counter plugin of Image J software (U.S. National Institutes of Health, Bethesda, Maryland, USA). Cells in mitosis were scored by visual inspection of pH3-positive cells.

### Statistical Analysis

Statistical analysis was carried out using SigmaStat (version 3.5; Systat Software Inc). Proportions were compared by z-test. Comparisons of mean values were made by z-test or by One Way ANOVA with pairwise analysis by Holm-Sidak test.

### Results

As shown in Figure 2, spina bifida occurred in approximately 15% of homozygous curly tail (ct/ct) embryos (controls). Maternal supplementation with a combination of thymidine + adenine (Thy + Ade) resulted in a significant reduction in the frequency of spina bifida (* p<0.01, z-test). There was also a trend towards reduced frequency of spina bifida among mice treated with thymidine + hypoxanthine (Thy + Hyp) or thymidine alone. These results were based on an analysis of 172 control embryos, 88 thy + ade embryos, 64 thy + hyp embryos, 60 ade only embryos, 74 thy only embryos. These results show that in vivo supplementation with a combination of thymidine and purine bases reduces the risk of spinal neural tube defects.

Embryos removed from pregnant females at E11.5-13.5 were scored for the outcome of low spinal neurulation as: normal (ST, straight tail), tail flexion defect (CT, curled tail), or open spina bifida which was always associated with a tail flexion defect (SB plus CT). Tail flexion defects arise when closure of the neural tube is delayed but does ultimately occur. Among curly tail embryos that do not exhibit spina bifida approximately half develop a tail flexion defect.

The frequency of spina bifida (Figure 3) was significantly lower among offspring of mice supplemented with Thy + Ade (n= 88 embryos) or Thy + GMP (n = 80) than among controls (n = 187 embryos; * p<0.01; z-test). 85% lower frequency of spina bifida was observed among embryos treated with thymidine + adenine than controls. There was a trend towards reduced spina bifida frequency among embryos exposed to Thy only (n = 74) or Thy + Hyp (n = 64) but this was non-significant, while Ade only (n = 60) had no effect.

In agreement with these findings, analysis of the data ona litter-by-litter basis showed that the mean proportion of embryos with spina bifida per litter was significantly lower fro mice treated with thymidine + adenine or thymidine + GMP than for controls (Figure 6A).

In addition to open spina bifida we analysed the frequency of tail flexion defects, which arise when neural tube closure is delayed, and straight tails, which occur among embryos in which spinal neurulation is apparently normal. Correlating with the data for spina bifida, the frequency of straight-tailed embryos, unaffected by either spina bifida or tail flexion defects, was significantly higher in the thymidine + adenine and thymidine + GMP groups (Fig. 3, Fig. 6B). There was also a higher frequency of unaffected (straight-tailed) embryos among offspring of mice treated with thymidine only, although this was not statistically significant in the litter-by-litter analysis (Fig. 6B).

There was a significantly higher frequency of straight tails among offspring of mice supplemented with Thy, Thy + Ade or Thy + GMP than among control litters (# p<0.01, ## p<0.001; z-test), correlating with reduced rates of spina bifida in these groups.

Combined supplementation thus produced a striking protective effect, with 85% lower frequency of spina bifida among embryos treated with thymidine + adenine than controls (Fig. 3). We also observed a significant protective effect of thymidine + GMP, but not thymidine + hypoxanthine (Fig. 3). In agreement with these findings, analysis of the data on a litter-by-litter basis showed that the mean proportion of embryos with spina bifida per litter was significantly lower for mice treated with thymidine + adenine and thymidine + GMP than for controls (results not shown).

The frequency of exencephaly (cranial NTDs) among genetically predisposed curly tail embryos was also assessed. The frequency of exencephaly was 6.8% among embryos from control litters. There was a trend towards diminished frequency of exencephaly among embryos treated with thymidine and adenine, thymidine and GMP, or thymidine only, compared with controls (3.0%, 2.5% and 2.7% respectively, see Figure 4).

In order to evaluate potential effects of treatment on litter size or embryonic viability, the number of embryos in each litter was recorded together with the presence of dead or resorbed embryos. As shown in Table 1 below, there was no statistically significant difference in the litter size or the number of dead/resorbed embryos per litter. This indicates that reduction in NTD frequency did not result from lethality of affected embryos.

To evaluate uptake of nucleotide precursors, mouse embryos were cultured from E9.5-10.5 in the presence of [³H]-thymidine, [³H]-adenine or [³H]-hypoxanthine. Detection of [³H]-labelling in genomic DNA (Table 2) showed that supplemental nucleotides cross the yolk sac and are incorporated into DNA in the embryo. These data also confirm that the salvage enzymes TK, APRT and HPRT (Fig. 1) are active at neurulation stages.

Cell proliferation was analysed at E10.5, at the axial level of the closing spinal neural folds. Mitotic index was determined at the level of the closure point of the neural folds at the posterior neuropore. Phospho-histone H3 (pH-H3)-positive cells were counted on transverse sections immediately anterior and posterior to the closure point.

A significant increase in the mitotic index was observed in the hindgut and neural folds of thymidine + adenine treated embryos compared with controls (Fig. 5A, *p < 0.05, t-test, n = 7 treated and 9 control embryos with 7-8 sections analysed per embryo). Importantly, there was a 1.63-fold increase in hindgut mitotic index, but only a 1.39-fold increase in neuroepithelial mitotic activity. This proportionally greater effect of nucleotide supplementation on hindgut proliferation is expected to minimise the dorsal-ventral growth imbalance that is known to hamper neural fold closure in *curly tail* embryos. Moreover, the increase in mitotic index we observed in the hindgut is similar to the 1.7-fold increase observed in *ct* embryos whose spina bifida was rescued by a *Grhl3* transgene. Taken together, these findings suggest that enhanced proliferation underlies the protective effect of supplemental nucleotides.

Measurement of embryo size at E13.5 (measurement of crown-rump length), following cessation of treatment at E10.5, did not reveal a long-term effect of treatment on overall growth (Fig. 5B).

**Table 1. Litter size and resorption rate is not affected by nucleotide supplementation.**

| | | | |
|---|---|---|---|
| The number of live embryos in each litter was recorded together, with the presence of any dead embryos or resorptions. Data are expressed as mean ± SEM. There was no statistically significant difference between treatments in the litter size or the number of dead/resorbed embryos per litter (One Way ANOVA). | | | |

| **Treatment** | **No. Litters** | **Mean litter size** | **Resorption per litter** |
|---|---|---|---|
| Control | 15 | 6.9 ± 0.5 | 0.6 ± 0.3 |
| Thy + Ade | 14 | 7.1 ± 0.4 | 0.2 ± 0.1 |
| Thy + Hyp | 8 | 8.0 ± 0.7 | 0.1 ± 0.1 |
| Thy + GMP | 8 | 8.9 ± 0.6 | 0.3 ± 0.2 |
| Ade only | 8 | 7.5 ± 0.7 | 0.8 ± 0.3 |
| Thy only | 10 | 7.4 ± 0.8 | 0.9 ± 0.4 |

**Table 2: Incorporation of exogenous nucleotide precursors into genomic DNA.**

| | | | |
|---|---|---|---|
| Embryos were cultured from E9.5-10.5 in the presence of tritiated compounds (n = 3 per treatment; doses indicated in Bq are equivalent to 1 or 2 µCi/ml). Labelling was normalised to the DNA content of the embryo and expressed as mean cpm/µg (± SEM). Based on measured cpm of nucleotide stock solutions an approximation of incorporation of [³H]-labelled molecule was calculated (for the 0.07 nmol/ml dose): thymidine, 18.7 fmol/µg DNA; hypoxanthine, 2.9 fmol/µg DNA; adenine 1.2 fmol/µg DNA. These data suggest that a greater amount of thymidine was incorporated into genomic DNA than adenine or hypoxanthine. However, the basal concentration of unlabelled molecules in the culture medium may have differed between nucleotides such that specific activity varied. | | | |

| **Treatment** | **Radioactivity (Bq/ml)** | **Amount of supplemental precursor (nmol/ml)** | **[³H] incorporation into genomic DNA (cpm/µg)** |
|---|---|---|---|
| Control | 0 | 0 | 6.3 ± 1.0 |
| [³H]-thymidine | 37,000 | 0.07 | 898.4 ± 98.0 |
| [³H]-hypoxanthine | 37,000 | 0.07 | 116.6 ± 9.3 |
| [³H]-adenine | 37,000 | 0.036 | 40.0 ± 6.6 |
| [³H]-adenine | 74,000 | 0.07 | 82.8 ± 6.7 |

## Claims

1. Thymidine or dTMP for use in a method of preventing or prophylactically treating a neural tube defect, wherein the thymidine or dTMP is administered in combination with one or more purine molecules selected from adenine, guanine, adenosine, guanosine, AMP and GMP.

2. Thymidine or dTMP for use according to claim 1, the method comprising administering the thymidine or dTMP to a female mammal that is pregnant or that may become pregnant.

3. Thymidine or dTMP for use according to claim 1 or 2, wherein the purine molecule is selected from adenine, adenosine and AMP.

4. Thymidine or dTMP for use according to claim 1 or 2, wherein the purine molecule is selected from guanine, guanosine and GMP.

5. Thymidine or dTMP for use according to any one of claims 1 to 4, wherein the thymidine or dTMP and the purine molecule are administered in a single composition.

6. Thymidine or dTMP for use according to any one of claims 1 to 4, wherein the thymidine or dTMP and the purine molecule are administered simultaneously, separately or sequentially.

7. Thymidine or dTMP for use according to any one of the preceding claims, wherein the method further comprises administering folic acid or folate.

8. Thymidine or dTMP for use according to any one of the preceding claims, wherein the thymidine or dTMP and said purine molecule and optionally said folic acid or folate are orally administered.

9. A composition comprising thymidine and/or dTMP for use in a method of preventing or prophylactically treating a neural tube defect, wherein said composition further comprises one or more purine molecules as defined in any one of claims 1, 3 and 4.

10. A composition for use according to claim 9, the method comprising administering said composition to a female mammal that is pregnant or that may become pregnant.

11. A composition for use according to claim 9 or 10, wherein said composition further comprises folic acid and/or folate.

12. Thymidine or dTMP for use according to any one of claims 1 to 8 or a composition for use according to any one of claims 9 to 11, the method comprising administering the thymidine or dTMP or composition to a female mammal that is pregnant or that may become pregnant, wherein
(a) said method is for preventing miscarriage in said female mammal; and/or
(b) said method is for preventing or treating recurrent miscarriage in said female mammal.

13. Thymidine or dTMP for use according to any one of claims 1 to 8 or 12 or a composition for use according to any one of claims 9 to 12, the method comprising administering the thymidine or dTMP or composition to a female mammal that is pregnant or that may become pregnant wherein said female mammal is non-responsive to treatment with folate or folic acid.

14. Thymidine or dTMP for use according to any one of claims 1 to 8, 12 or 13 or a composition for use according to any one of claims 9 to 13, the method comprising administering the thymidine or dTMP or composition to a female mammal that is pregnant or that may become pregnant, wherein
(a) said female mammal has previously given birth to an offspring having a neural tube defect;
(b) said female mammal has previously miscarried an offspring having a neural tube defect; or
(c) said female mammal has been diagnosed as susceptible to or at risk of producing offspring having a neural tube defect.

## Patentansprüche

1. Thymidin oder dTMP zur Verwendung in einem Verfahren des Verhinderns oder prophylaktischen Behandelns eines Neuralrohrdefekts, wobei das Thymidin oder dTMP in Kombination mit einem oder mehreren Purinmolekülen verabreicht wird, die aus Adenin, Guanin, Adenosin, Guanosin, AMP und GMP ausgewählt sind.

2. Thymidin oder dTMP zur Verwendung nach Anspruch 1, wobei das Verfahren das Verabreichen des Thymidin oder dTMP an ein weibliches Säugetier, das trächtig ist oder trächtig werden könnte, umfasst.

3. Thymidin oder dTMP zur Verwendung nach Anspruch 1 oder 2, wobei das Purinmolekül aus Adenin, Adenosin und AMP ausgewählt wird.

4. Thymidin oder dTMP zur Verwendung nach Anspruch 1 oder 2, wobei das Purinmolekül aus Guanin, Guanosin und GMP ausgewählt wird.

5. Thymidin oder dTMP zur Verwendung nach einem der Ansprüche 1 bis 4, wobei das Thymidin oder dTMP und das Purinmolekül in einer einzigen Zusammensetzung verabreicht werden.

6. Thymidin oder dTMP zur Verwendung nach einem der Ansprüche 1 bis 4, wobei das Thymidin oder dTMP und das Purinmolekül gleichzeitig, getrennt oder nacheinander verabreicht werden.

7. Thymidin oder dTMP zur Verwendung nach einem der vorstehenden Ansprüche, wobei das Verfahren ferner das Verabreichen von Folsäure oder Folat umfasst.

8. Thymidin oder dTMP zur Verwendung nach einem der vorstehenden Ansprüche, wobei das Thymidin oder dTMP und das Purinmolekül und optional die Folsäure oder das Folat oral verabreicht werden.

9. Zusammensetzung, die Thymidin und/oder dTMP zur Verwendung in einem Verfahren des Verhinderns oder prophylaktischen Behandelns eines Neuralrohrdefekts umfasst, wobei die Zusammensetzung ferner ein oder mehrere Purinmoleküle nach einem der Ansprüche 1, 3 und 4 umfasst.

10. Zusammensetzung zur Verwendung nach Anspruch 9, wobei das Verfahren das Verabreichen der Zusammensetzung an ein weibliches Säugetier, das trächtig ist oder trächtig werden könnte, umfasst.

11. Zusammensetzung nach Anspruch 9 oder 10, wobei die Zusammensetzung ferner Folsäure oder Folat umfasst.

12. Thymidin oder dTMP zur Verwendung nach einem der Ansprüche 1 bis 8 oder Zusammensetzung zur Verwendung nach einem der Ansprüche 9 bis 11, wobei das Verfahren das Verabreichen des Thymidin oder dTMP oder der Zusammensetzung an ein weibliches Säugetier, das trächtig ist oder trächtig werden könnte, umfasst, wobei
(a) das Verfahren dem Verhindern einer Fehlgeburt bei dem weiblichen Säugetier dient; und/oder
(b) das Verfahren dem Verhindern oder Behandeln von wiederkehrender Fehlgeburt in dem weiblichen Säugetier umfasst.

13. Thymidin oder dTMP zur Verwendung nach einem der Ansprüche 1 bis 8 oder 12 oder Zusammensetzung zur Verwendung nach einem der Ansprüche 9 bis 12, wobei das Verfahren das Verabreichen des Thymidin oder dTMP oder der Zusammensetzung an ein weibliches Säugetier, das trächtig ist oder trächtig werden könnte, umfasst, wobei das weibliche Säugetier nicht auf die Behandlung mit Folat oder Folsäure anspricht.

14. Thymidin oder dTMP zur Verwendung nach einem der Ansprüche 1 bis 8, 12 oder 13 oder Zusammensetzung zur Verwendung nach einem der Ansprüche 9 bis 13, wobei das Verfahren das Verabreichen des Thymidin oder dTMP oder der Zusammensetzung an ein weibliches Säugetier, das trächtig ist oder trächtig werden könnte, umfasst, wobei
(a) das weibliche Säugetier zuvor Nachwuchs geboren hat, der einen Neuralrohrdefekt hat;
(b) das weibliche Säugetier zuvor eine Fehlgeburt hatte mit Nachwuchs, der einen Neuralrohrdefekt hatte; oder
(c) das weibliche Säugetier als anfällig oder gefährdet diagnostiziert wurde, Nachwuchs zu bekommen, der einen Neuralrohrdefekt hat.

## Revendications

1. Thymidine ou dTMP destinée à une utilisation ayant pour but de prévenir ou de traiter de manière prophylactique une anomalie d'un tube neural, ladite thymidine ou dTMP étant administrée en combinaison avec une ou plusieurs molécules puriques choisies parmi l'adénine, la guanine, l'adénosine, la guanosine, l'AMP, et la GMP.

2. Thymidine ou dTMP destinée à une utilisation selon la revendication 1, la méthode comprenant l'administration de la thymidine ou de la dTMP à un mammifère femelle gravide ou susceptible de devenir gravide.

3. Thymidine ou dTMP destinée à une utilisation selon la revendication 1 ou 2, dans laquelle la molécule purique est choisie parmi l'adénine, l'adénosine et l'AMP.

4. Thymidine ou dTMP destinée à une utilisation selon la revendication 1 ou 2, dans laquelle la molécule purique est choisie parmi la guanine, la guanosine et la GMP.

5. Thymidine ou dTMP destinée à une utilisation selon l'une quelconque des revendications 1 à 4, ladite thymidine ou dTMP et la molécule purique étant incorporées dans une unique composition.

6. Thymidine ou dTMP destinée à une utilisation selon l'une quelconque des revendications 1 à 4, ladite thymidine ou dTMP et la molécule purique étant administrées simultanément, séparément ou séquentiellement.

7. Thymidine ou dTMP destinée à une utilisation selon l'une quelconque des revendications précédentes, la méthode comprenant en outre l'administration d'un acide folique ou d'un folate.

8. Thymidine ou dTMP destinée à une utilisation selon l'une quelconque des revendications précédentes, lesdites thymidine ou dTMP et ladite molécule purique et éventuellement ledit acide folique ou folate étant administrés oralement.

9. Composition comprenant la thymidine et/ou la dTMP destinée à une utilisation dans une méthode ayant pour but de prévenir ou de traiter de manière prophylactique une anomalie d'un tube neural, ladite composition comprenant en outre une ou plusieurs molécules puriques telles que définies dans l'une quelconque des revendications 1, 3 et 4.

10. Composition destinée à une utilisation selon la revendication 9, la méthode comprenant l'administration de ladite composition à un mammifère femelle gravide ou susceptible de devenir gravide.

11. Composition destinée à une utilisation selon la revendication 9 ou 10, ladite composition comprenant en outre un acide folique et/ou un folate.

12. Thymidine ou dTMP destinée à une utilisation selon l'une quelconque des revendications 1 à 8 ou composition destinée à une utilisation selon l'une quelconque des revendications 9 à 11, la méthode comprenant l'administration de la thymidine ou de la dTMP ou de la composition à un mammifère femelle gravide ou susceptible de devenir gravide,
(a) ladite méthode ayant pour but de prévenir un avortement spontané chez ledit mammifère femelle ; et/ou
(b) ladite méthode ayant pour but de prévenir ou de traiter les avortements spontanés à répétition chez ledit mammifère femelle.

13. Thymidine ou dTMP destinée à une utilisation selon l'une quelconque des revendications 1 à 8 ou composition destinée à une utilisation selon l'une quelconque des revendications 9 à 12, la méthode comprenant l'administration de la thymidine ou de la dTMP ou de la composition à un mammifère femelle gravide ou susceptible de devenir gravide, ledit mammifère femelle étant non réactif au traitement au folate ou à l'acide folique.

14. Thymidine ou dTMP destinée à une utilisation selon l'une quelconque des revendications 1 à 8, 12 ou 13 ou composition destinée à une utilisation selon l'une quelconque des revendications 9 à 13, la méthode comprenant l'administration de la thymidine ou de la dTMP ou de la composition à un mammifère femelle gravide ou susceptible de devenir gravide,
(a) ledit mammifère femelle ayant préalablement donné naissance à une progéniture présentant une anomalie d'un tube neural ;
(b) ledit mammifère femelle ayant préalablement avorté spontanément d'une progéniture présentant une anomalie d'un tube neural ; ou
(c) ledit mammifère femelle ayant été diagnostiqué susceptible ou risquant de donner naissance à une progéniture présentant une anomalie d'un tube neural.
